# EUROPÄISCHE PATENTANMELDUNG

(11) **EP 3 272 289 A1**
(43) Veröffentlichungstag der Anmeldung: **24.01.2018**
(21) Anmeldenummer: 17172730.8
(22) Anmeldetag: 24.05.2017
(51) Int. Cl.: A61B 6/04, A61G 7/10, A61G 13/10, A61B 5/055

(54) **ANORDNUNG FÜR PATIENTENLAGERUNGSTISCHE**

(30) Priorität: 06.06.2016 AT 1242016 U
(71) Anmelder: Kapun, Christian, 9061 Klagenfurt am Wörthersee (AT)
(72) Erfinder: Kapun, Christian, 9061 Klagenfurt am Wörthersee (AT)
(74) Vertreter: Beer & Partner Patentanwälte KG

(57) **Zusammenfassung**

Eine Anordnung (1), die das Umlagern von Patienten von einem Patiententransportwagen auf den Patientenlagerungstisch (2) einer bildgebenden Untersuchungseinrichtung erleichtert, umfasst eine, am fußseitigen Ende des Patientenlagerungstisches (2) festzulegende Einrichtung (4) mit Verriegelungen (9) für das Festlegen des Patiententransportwagens. Weiters sind eine auf den Patientenlagerungstisch (2) aufzulegende Aufnahmeplatte (3) und ein zwischen der Einrichtung (4) und der Aufnahmeplatte (3) angeordneter Aufnahmeteil (6) vorgesehen. Die Aufnahmeplatte (3) und der Aufnahmeteil (6) sind mit Rollen (7) und (8) bestückt, sodass eine Platte (5) zum Lagern eines Patienten (5), auf der ein Patient fixiert ist, vom Patiententransportwagen auf den Rollen (7) und (8) rollend auf den Patientenlagerungstisch (4) und zur bildgebenden Untersuchungseinrichtung hingeschoben, also umgelagert werden kann, ohne dass der Patient von der Platte (5) zum Lagern von Patienten des Patiententransportwagens abgehoben werden muss.

## Beschreibung

Die Erfindung betrifft eine Anordnung zum Anbringen an Patientenlagerungstische von bildgebenden Einrichtungen für die Untersuchung von Patienten.

Einrichtungen für bildgebende Verfahren zum Untersuchen von Personen und Patienten, wie Computertomographie, Röntgen, Magnetresonanztomografie, Angiographie usw., umfassen einen Patientenlagerungstisch, auf dem die zu untersuchende Person (Patient) liegt.

Das Ergebnis der Untersuchung bei mehrfach verletzten, polytraumatisierten Patienten und das Ergebnis der Behandlung solcher Patienten wird ganz wesentlich vom reibungslosen Aufeinandertreffen der Arbeitsabläufe im Bereich der Primärversorgung beeinflusst. Dies erfordert neben dem Stabilisieren der Vitalparameter des Patienten eine rasche Diagnostik und Therapie vitalbedrohlicher Verletzungen. Bei mehrfachverletzten Patienten, insbesondere bei Wirbelverletzungen, stellt jede Umlagerung des Patienten das Risiko einer dadurch verursachten Verschlimmerung der ursprünglichen Verletzung und von deren Folgen dar.

Aus diesem Grund gibt es verschiedene Vorschläge für den Transport und die Lagerung, mit welchen Patienten nach Beendigung einer Operation auf einer Transportplatte fixiert werden. Um den Patienten zusammen mit einer Platte, auf welcher der Patient gelagert ist, an Einrichtungen für bildgebende Verfahren, wie Computertomographie, Röntgen, Magnetresonanztomografie, Angiographie usw., umlagern zu können, besteht Bedarf an einer einfachen Anordnung, mit der Patienten von Patiententransportwagen auf Platten zum Lagern von Patienten von Einrichtungen für medizinische, bildgebende Verfahren umgelagert werden können.

Der Erfindung liegt die Aufgabe zugrunde, einen Anordnung der eingangs genannten Gattung zur Verfügung zu stellen, die ein sicheres und problemloses Umlagern von Patienten von einem Patiententransporter (Transportwagen) auf Einrichtungen zur bildgebenden Untersuchung erlaubt.

Gelöst wird diese Aufgabe erfindungsgemäß mit einer Anordnung, welche die Merkmale von Anspruch 1 aufweist.

Bevorzugte und vorteilhafte Ausgestaltungen der erfindungsgemäßen Anordnung sind Gegenstand der Unteransprüche.

Die Erfindung stellt ein abnehmbares, röntgenstrahlentransparentes und antimagnetisches Rollensystem zur Verfügung, das auf der Tischplatte eines Patientenlagerungstisches von Einrichtungen zum Ausführen bildgebender Verfahren angebracht werden kann.

Da die erfindungsgemäße Anordnung vom Patientenlagerungstisch ohne Weiteres auch von nur einer Person abgenommen werden kann, können auch normale Untersuchungen von Patienten, die keine besondere Sorgfalt beim Umlagern von Patienten von Patiententransportwagen auf Patientenlagerungstische bildgebender Einrichtungen erfordern, ausgeführt werden.

Da bei der erfindungsgemäßen Anordnung eine Aufnahmeplatte vorgesehen ist, die mit Rollen bestückt ist, kann eine Platte zum Lagern von Patienten von einem Patiententransportwagen ohne Weiteres auf den Rollen auf einen Patientenlagerungstisch verschoben werden, ohne dass der Patient von der Platte zum Lagern von Patienten des Patiententransportwagens abgehoben und auf einen Patientenlagerungstisch einer bildgebenden Einrichtung umgelagert werden muss.

In ihrer grundsätzlichen Ausführungsform umfasst die erfindungsgemäße Anordnung eine mit Rollen bestückte Aufnahmeplatte, die auf einem Patientenlagerungstisch lösbar angebracht (aufgelegt) werden kann.

Des Weiteren umfasst die erfindungsgemäße Anordnung in ihrer grundsätzlichen Ausführungsform eine an einem Ende (z.B. dem Fußende) des Patientenlagerungstisches lösbar festlegbare Einrichtung zum Festlegen eines Patiententransportwagens, auf dem eine Platte zum Lagern von Patienten angeordnet ist.

Insbesondere ist die Einrichtung zum Festlegen eines Patiententransportwagens am Fußende des Patientenlagerungstisches angeordnet. So wird während der Übergabe der Platte zum Lagern von Patienten vom Patiententransportwagen auf den Patientenlagerungstisch der bildgebenden Einrichtung eine sichere und stabile Verankerung des Patiententransportwagens an dem Patientenlagerungstisch gewährleistet.

Weitere Einzelheiten und Merkmale der erfindungsgemäßen Anordnung und deren Verwendung ergeben sich aus der nachstehenden Beschreibung unter Bezugnahme auf die Zeichnungen.

Es zeigt:

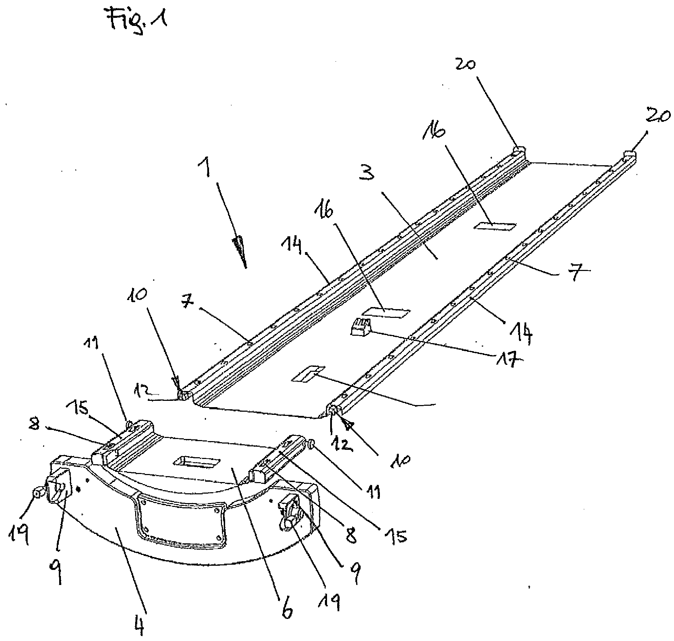

- Fig. 1: in auseinandergezogener Darstellung die Teile einer erfindungsgemäßen Anordnung und
- Fig. 2: die erfindungsgemäße Anordnung an einem Patientenlagerungstisch angebracht.

Eine erfindungsgemäße Anordnung 1, die an einem Patientenlagerungstisch 2 anzubringen ist, umfasst eine Aufnahmeplatte 3, die auf den Patientenlagerungstisch 2 aufgelegt wird.

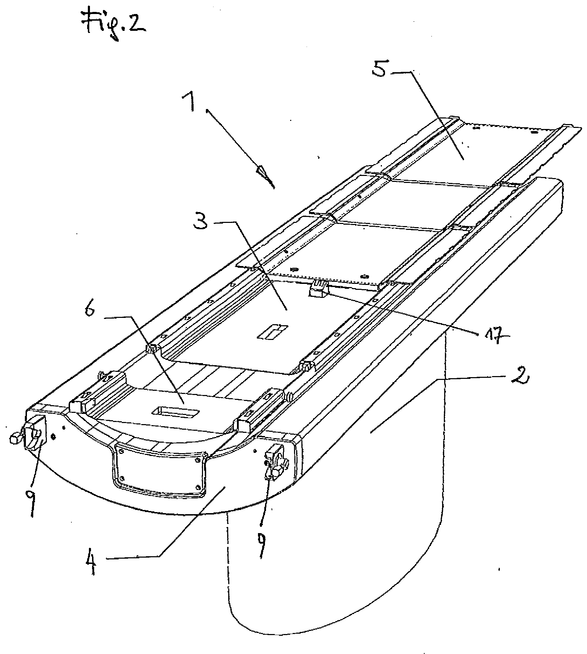

Am Fußende des Patientenlagerungstisches 2 wird eine Einrichtung 4 zum Festlegen eines (nicht gezeigten) Patiententransportwagens befestigt. Zwischen der Einrichtung 4 zum Festlegen eines Patiententransportwagens und der Aufnahmeplatte 3 ist ein Aufnahmeteil 6 vorgesehen, der ebenfalls im Bereich des Fußendes des Patientenlagerungstisches 2 aufgelegt wird. Die Aufnahmeplatte 3 umfasst Rollen 7. Auch der Aufnahmeteil 6 umfasst Rollen 8. Die Rollen 7 und 8 sind jeweils im Bereich der Längsränder 14 bzw. 15 der Aufnahmeplatte 3 und des Aufnahmeteils 6 angeordnet. Die Rollen 7 und 8 sind an der Unterseite der Aufnahmeplatte 3 und an der Unterseite des Aufnahmeteils 6 angeordnet und ragen durch Löcher in der Aufnahmeplatte 3 und den Aufnahmeteil 6 nach oben, sodass eine Platte 5 zum Lagern eines Patienten (Fig. 2) auf der erfindungsgemäßen Anordnung 1 leicht verschoben werden kann, insbesondere von einem Patiententransportwagen auf die Anordnung 1 verschoben werden kann.

In der Einrichtung 4 zum Festlegen eines Patiententransportwagens sind Verriegelungseinrichtungen 9 zum Festlegen des Patiententransportwagens 1 am Patientenlagerungstisch 2 vorgesehen. Zum Betätigen der Verriegelung 9 sind Hebel 19 vorgesehen.

Es ist darauf hinzuweisen, dass sowohl die Aufnahmeplatte 3, der Aufnahmeteil 6 als auch die Rollen 7 und 8 aus strahlendurchlässigem Werkstoff gefertigt sind. Wenn die erfindungsgemäße Anordnung 1 für bildgebende Verfahren mit magnetischen Feldern, z.B. Magnetresonanztomografie, verwendet wird, sind die Aufnahmeplatte 3, der Aufnahmeteil 6 und die Rollen 7 und 8 aus antimagnetischem Werkstoff gefertigt.

Um die Aufnahmeplatte 3 von dem Patientenlagerungstisch 2 leicht entfernen zu können, wenn die erfindungsgemäße Anordnung 1 nicht benötigt wird, sind in ihr Griffmulden 16 vorgesehen.

An der Aufnahmeplatte 3 ist weiters eine Verriegelung 17 vorgesehen, die dazu dient, eine Patientenlagerungsplatte 5 zu fixieren, wenn diese auf dem Patientenlagerungstisch 2 in Richtung auf die Untersuchungseinrichtung hin verschoben worden ist. So kann eine zu untersuchende Person (Patient), der auf der Platte 5 fixiert ist, verschoben werden, wobei die Sicherheit des Patienten und die artefaktfreie Untersuchung gewährleistet ist.

Die Aufnahmeplatte 3 wird an dem auf dem Patientenlagerungstisch 2 befestigten Aufnahmeteil 6 mit Hilfe von Verriegelungen 10 lösbar festgelegt. Die Verriegelungen 10 umfassen am dem Aufnahmeteil 6 angeordnet je einen Verriegelungsbolzen 11. Die Verriegelungsbolzen 11 sind quer zur Längserstreckung des Aufnahmeteils 6 verschiebbar. Am Rand der Aufnahmeplatte 3 sind Bolzenaufnahmen 12 vorgesehen. Durch Herausziehen der Verriegelungsbolzen 11 können die Verriegelungen 10 zwischen der Aufnahmeplatte 3 und dem Aufnahmeteil 6 gelöst werden.

An dem vom Aufnahmeteil 6 abgekehrten Ende der Aufnahmeplatte 3 sind an deren Längsrändern 14 Anschläge 20 vorgesehen, die den Verschiebebereich einer Platte 5 zum Lagern von Patienten auf der Anordnung 1 begrenzen.

Zusammenfassend kann ein Ausführungsbeispiel der erfindungsgemäßen Anordnung 1 wie folgt beschrieben werden:
Eine Anordnung 1, die das Umlagern von Patienten von einem Patiententransportwagen auf den Patientenlagerungstisch 2 einer bildgebenden Untersuchungseinrichtung erleichtert, umfasst eine am fußseitigen Ende des Patientenlagerungstisches 2 festzulegende Einrichtung 4 mit Verriegelungen 9 für das Festlegen des Patiententransportwagens. Weiters sind eine auf den Patientenlagerungstisch 2 aufzulegende Aufnahmeplatte 3 und ein zwischen der Einrichtung 4 und der Aufnahmeplatte 3 angeordneter Aufnahmeteil 6 vorgesehen. Die Aufnahmeplatte 3 und der Aufnahmeteil 6 sind mit Rollen 7 und 8 bestückt, sodass eine Platte 5 zum Lagern eines Patienten, auf der ein Patient fixiert ist, vom Patiententransportwagen auf den Rollen 7 und 8 rollend auf den Patientenlagerungstisch 4 und zur bildgebenden Untersuchungseinrichtung hingeschoben, also umgelagert werden kann, ohne dass der Patient von der Platte 5 zum Lagern von Patienten des Patiententransportwagens abgehoben werden muss.

## Patentansprüche

1. Anordnung (1) zum Anbringen an Patientenlagerungstische (2) von bildgebenden Einrichtungen für die Untersuchung von Patienten, **gekennzeichnet durch** eine auf dem Patientenlagerungstisch (2) lösbar anzubringende und mit Rollen (7) bestückte Aufnahmeplatte (3) für eine Platte (5) zum Lagern von Patienten, wobei die Aufnahmeplatte (3) und die Rollen (7) aus strahlendurchlässigem und/oder antimagnetischem Werkstoff bestehen, und durch eine an einem Ende des Patientenlagerungstisches (2) lösbar festlegbare Einrichtung (4) zum Festlegen eines Patiententransportwagens, auf dem eine Platte (5) zum Lagern von Patienten angeordnet ist.

2. Anordnung nach Anspruch 1, **dadurch gekennzeichnet, dass** zwischen der Aufnahmeplatte (3) für die Platte (5) zum Lagern von Patienten und der Einrichtung (4) zum Festlegen eines Patiententransportwagens ein im Bereich eines Endes des Patientenlagerungstisches (2) festlegbarer, mit Rollen (8) bestückter Aufnahmeteil (6), der einschließlich der Rollen (8) aus strahlendurchlässigem und/oder antimagnetischem Werkstoff besteht, vorgesehen ist.

3. Anordnung nach Anspruch 1 oder 2, **dadurch gekennzeichnet, dass** eine Verriegelungseinrichtung (10) zum lösbaren Verbinden der Aufnahmeplatte (3) mit dem Aufnahmeteil (6) vorgesehen ist.

4. Anordnung nach Anspruch 3, **dadurch gekennzeichnet, dass** die Verriegelung (10) Verriegelungsbolzen (11) an dem Aufnahmeteil (6) und Bolzenaufnahmen (12) an der Aufnahmeplatte (3) umfasst.

5. Anordnung nach einem der Ansprüche 3 bis 4, **dadurch gekennzeichnet, dass** an der Einrichtung (4) Verriegelungen (9) mit Betätigungshebeln (19) zum Festlegen eines Patiententransportwagens vorgesehen sind.

6. Anordnung nach einem der Ansprüche 1 bis 5, **dadurch gekennzeichnet, dass** auf der Aufnahmeplatte (3) eine Verriegelung (17) zum Verbinden der Platte (5) zum Lagern von Patienten mit der Aufnahmeplatte (3) vorgesehen ist.

7. Anordnung nach einem der Ansprüche 1 bis 6, **dadurch gekennzeichnet, dass** in der Aufnahmeplatte (3) Griffe, insbesondere Griffmulden (16), vorgesehen sind.

8. Anordnung nach einem der Ansprüche 1 bis 7, **dadurch gekennzeichnet, dass** die Rollen (7, 8) der Aufnahmeplatte (3) und/oder des Aufnahmeteils (6) im Bereich der Längsränder (14, 15) derselben vorgesehen sind.

9. Anordnung nach einem der Ansprüche 1 bis 8, **dadurch gekennzeichnet, dass** die Rollen (7, 8) unterhalb der Aufnahmeplatte (3) und/oder des Aufnahmeteils (6) gelagert sind und durch Löcher in denselben auf deren Oberseite ragen.
